**Europäisches Patentamt**

**European Patent Office**

**Office européen des brevets**

(19)

(11) Veröffentlichungsnummer : **0 465 866 A1**

(12) **EUROPÄISCHE PATENTANMELDUNG**

(21) Anmeldenummer : **91109822.6**

(22) Anmeldetag : **14.06.91**

(51) Int. Cl.$^5$ : **A61B 17/60**

(30) Priorität : **15.06.90 DE 9006716 U**

(43) Veröffentlichungstag der Anmeldung :
**15.01.92 Patentblatt 92/03**

(84) Benannte Vertragsstaaten :
**AT BE CH DE DK ES FR GB GR IT LI LU NL SE**

(71) Anmelder : **Schewior, Thomas, Dr.med.**
**Lessingstrasse 1**
**W-6903 Neckargemünd (DE)**

(72) Erfinder : **Schewior, Thomas, Dr.med.**
**Lessingstrasse 1**
**W-6903 Neckargemünd (DE)**

(74) Vertreter : **Mierswa, Klaus, Dipl.-Ing.**
**Friedrichstrasse 171**
**W-6800 Mannheim 24 (DE)**

(54) **Draht oder Nagel mit Haltemittel, vorzugsweise für einen Fixateur.**

(57) Die Erfindung betrifft einen Draht (1) oder
Nagel (28) mit Haltemittel (14,33), insbesondere
für einen Fixateur zum Einrichten, Befestigen
und Regulieren der Spannlage von Knochenabschnitten, wie Kirschner-Draht oder Zügeldraht
oder Steinmann-Nagel, mit einem Gewinde- und/oder Halterungsteil an beiden Enden zum
Halten des Drahtes oder Nagels innerhalb mindestens eines Bolzens (22) des Fixateurs mittels
Muttern (19) und/oder der Haltemitteln (14,33),
die auf die Enden des Drahtes oder Nagels
lösbar aufgesetzt sind. Der Draht (1) oder Nagel
(28) besitzt einen glatten Schaft (5,30), der am
penetrierenden Ende einen Bohrkopf (9,32) aufweist, an den sich ein gegenüber dem Schaft
verjüngtes Teil (6,31) mit planparallelen Begrenzungsflächen (12, 12') anschließt, dessen Breite
(b) geringer ist als der Durchmesser (d) des
Schaftes. Das Haltemittel (14,33) weist einen
durchgehenden Schlitz (16,34) auf, der breiter
ist als die Breite (6) des verjüngtenTeils (6,31)
des Schaftes (5,30), wobei die Länge des Haltemittels (14,33) kleiner ist als die Länge (1) des
verjüngten Teils des Schaftes zum Aufsetzen
des Haltemittels (14,33) auf den verjüngten Teil
(6,31) des Schaftes (5,30). Innerhalb des Haltemittels (14,33) ist zentrisch in Richtung des
Schlitzes (16,34) von dem dem penetrierenden
Ende (15) gegenüberliegenden Ende des Haltemittels (14,33) ausgehend mindestens eine
Sackbohrung (17,35,35') angeordnet, deren
Durchmesser größer ist als der Durchmesser (d)
des Schaftes (5,30), wobei in Zugstellung des
Drahtes (1) oder Nagels (28) innerhalb des Fixateurs der Bohrkopf (9,32) desselben vollständig
innerhalb des Haltemittels (14,33) aufgenommen ist.

Fig. 1

EP 0 465 866 A1

**Technisches Gebiet:**

Die Erfindung betrifft einen Draht oder Nagel mit Haltemittel, insbesondere für einen Fixateur, gemäß dem Oberbegriff des Patentanspruchs 1.

**Stand der Technik:**

Durch die EP-A 3-0146872 ist eine Vorrichtung zum Einrichten von Knochenabschnitten und/oder Knochenfragmenten bekanntgeworden, die aus durch Stangen und Befestigungsmittel in ein- und verstellbaren Abständen miteinander verbindbaren Stellringen und aus Spanndrähten besteht, die sich in den von den Stellringen aufgespannten Ebenen erstrecken und mit ihren Enden mittels Halterungen an voneinander entfernten Teilen jeweils eines Stellrings unter Längsspannung befestigbar sind. Der Befestigung der Spanndrahtenden an den Stellringen dienen nachstellbare Spannelemente.

Durch die WO 8810099 ist ein Ringfixateur zum Einrichten, Befestigen und Regulieren der Spannlage von Knochenabschnitten bekanntgeworden, der zur Erzielung von formverwandelnden Montagen nach allen Freiheitsgraden des Raumes aus Ringen besteht, deren Wandquerschnitte hochformatig oder quadratisch sind und die aus undurchbrochen wickelbaren, in Kunststoff-Matrix bettbaren Fasern ausgebildet sind, mit Drähten mit durchgehenden Gewinden zum Aufschrauben von befestigenden und spannungsregulierenden Spann- und zügelnden Positionsmuttern, Reitern und Haltebolzen, ebenso wie mit auswechselbaren Rahmenabschnitten, bestehend jeweils aus zwei parallel zueinander verlaufenden Rahmenstangen. Die Halterung der Spanndrähte in den dafür vorgesehenen Halterungen ist jedoch nicht optimal und ermöglicht insbesondere nicht ein freies Auswechseln von Drähten oder Nägeln, ohne nicht eine Mehrzahl von Verbindungen des Fixateurs lösen zu müssen.

Die transossären Enden der Drähte oder der Nägel bergen eine große Verletzungsgefahr in sich, die leicht zu Infektionen des Operateurs führen können.

**Aufgabe der Erfindung:**

Der Erfindung liegt die Aufgabe zugrunde, einen Draht oder Nagel mit Haltemittel, wie Kirschner-Draht oder Zügeldraht oder Steinmann-Nagel, insbesondere für einen Fixateur zum Einrichten, Befestigen, Regulieren und Zügeln der Spannlage von Kochenabschnitten, derart weiter zu gestalten, daß der Draht oder Nagel, auch unter Verspannung des Fixateurs, bei Vermeiden einer Verletzungsgefahr des Operateurs oder des Personals einfach gehandhabt und praktisch unter beliebigen Bedingungen ausgewechselt und manövriert werden kann, wobei die der Verspannung dienenden Draht- oder Nagelgewinde derart gestaltet sein sollen, daß diese nicht durch Weichteile gehen, damit Nerven, Sehnen und Blutgefäße nicht verletzt werden; ebenso soll der mit den Drähten oder Nägeln ausgestattete Fixateur einfach manövriert werden können.

**Darstellung der Erfindung und deren Vorteile:**

Die Aufgabe wird erfindungsgemäß gelöst durch folgende Merkmale:
a) der Draht oder Nagel besitzt einen glatten Schaft, der am penetrierenden Ende einen Bohrkopf aufweist, an den sich ein gegenüber dem Schaft verjüngtes Teil mit planparallelen Begrenzungsflächen anschließt, dessen Breite (b) geringer ist als der Durchmesser (d) des Schaftes
b) das Haltemittel weist einen durchgehenden Schlitz auf, der breiter ist als die Breite (6) des verjüngten Teils des Schaftes, wobei die Länge des Haltemittels kleiner ist als die Länge (1) des verjüngten Teils des Schaftes zum Aufsetzen des Haltemittels auf den verjüngten Teil des Schaftes
c) innerhalb des Haltemittels ist zentrisch in Richtung des Schlitzes von dem dem penetrierenden Ende gegenüberliegenden Ende des Haltemittels ausgehend mindestens eine Sackbohrung angeordnet, deren Durchmesser größer ist als der Durchmesser (d) des Schaftes
d) in Zugstellung des Drahtes oder Nagels innerhalb des Fixateurs ist der Bohrkopf vollständig innerhalb des Haltemittels aufgenommen.Weitere vorteilhafte Ausgestaltungen der Erfindung sind in den Unteransprüchen gekennzeichnet. Der erfindungsgemäße Draht oder Nagel besitzt den hervorstechenden Vorteil, daß der Bohrkopf des penetrierenden Drahtes oder Nagels immer im Haltemittel vollständig aufgenommen ist, so daß weder die Spitze, noch Schneiden des Bohrkopfes aus dem Haltemittel herausragen, welches als Schutzkappe wirkt. Dadurch wird zum ersten Mal nach dem Bohren und Aufsetzen des Haltemittels auf den transossären Draht oder Nagel die Verletzungsgefahr des Operateurs oder des Personals erheblich herabgesetzt, wodurch die Infektionsgefahr entscheidend gemindert wird. Das Haltemittel bewirkt somit in vorteilhafter Weise einen Schutz vor Infektionsgefahren. Des weiteren wird das Haltemittel beim Aufsetzen oder Abnehmen auf den Draht oder Nagel immer senkrecht zur Spitze oden den Schneiden des Bohrkopfes, d.h. seitlich, bewegt, also immer weg von der Richtung der größtmöglichen Verletzungsgefahr.

Aufgrund des glatten Schaftes des Drahtes oder des Nagels, der längenmäßig den Hauptteil der gesamten Länge ausmacht, besitzt derselbe den weiteren Vorteil, daß beim Durchbohren des Gewebes oder des Knochens keine Gewebeteile sich um den Schaft herumschlingen können. Das nur am nichtpenetrierenden Ende vorhandene Gewinde des Drahtes oder Nagels tritt nicht in die Weichteile ein, es ist jedoch so lang, daß genügend Spannweg vorhanden ist. Der Bohrkopf besitzt den Vorteil, daß derselbe aufgrund der zueinander geneigten, bohrartigen und scharfkantigen Schneiden eine gleichmäßige Materialentnahme innerhalb eines zu penetrierenden Knochens ermöglicht, wobei durch die Kanäle, die innerhalb des Bohrkopfes angeordnet sein können, der Abrieb des Knochens in die aufgrund der planparallelen Flächen des verjüngten Teils gebildeten Ausnehmungen des Schaftes gefördert wird.

In vorteilhafter Weise kann das lösbare Haltemittel am penetrierenden Ende des Drahtes oder Nagels ein Hohlkörper, wie Hohlzylinder oder eine Olive sein, deren Gestaltung mit dem verjüngten Teil des Schaftes des Drahtes oder Nagels in funktionaler Weise zusammenspielt, dergestalt, daß der Draht oder Nagel nach dem Aufsetzen des Haltemittels auf denselben spitzennah und verdrehungssicher gehaltert ist. Nach dem Spannen des Drahtes oder des Nagels ist derselbe drehfest und gegen eine Verschiebung fest gehaltert. Von weiterem Vorteil ist, daß der Draht oder Nagel jederzeit leicht ausgewechselt werden kann, wenn beispielsweise dessen Lage oder Richtung verändert werden muß. Unter Beibehaltung der Verspannung der übrigen Teile des Fixateurs ist es aufgrund der Konstruktion des Drahtes oder Nagels zusammen mit dem lösbaren Haltemittel am penetrierenden Ende möglich, den Draht oder Nagel leicht aus der Verankerung eines Bolzens oder aus seiner Zügelstellung direkt im Körper herauszunehmen, zu versetzen und wieder zu verankern oder nur den Winkel innerhalb des Bolzens zu verändern. Bei diesem Verankerungsprozeß sind keine schraubenden oder drehenden Bewegungen notwendig, sondern nur ein Aufsetzen des Haltemittels auf den verjüngten Teil des Drahtes oder Schaftes und ein axiales Verschieben des Haltemittels in Richtung zum Bohrkopf oder des Drahtes. Der Bohrkopf und der verjüngte Teil des Schaftes sowie das Gewindeteil bilden die Abschnitte des Drahtes oder Nagels, die nach der Penetration des Knochens und der Weichteile aus denselben herausragen und innerhalb der entsprechenden Halterungen des Fixateurs gehaltert sind.

In vorteilhafter Weise kann das Haltemittel für das penetrierende Ende des Drahtes auch eine Olive oder ein Stopper sein; in diesem Fall kann vorteilhafterweise der Draht ein Zügeldraht sein. Die Olive kann gleich ausgestaltet sein wie der Hohlzylinder bei einem Kirschner-Draht oder Steinmann-Nagel. Darüber hinaus kann in Richtung des durchgehenden Schlitzes innerhalb der Olive oder desStoppers von beiden Seiten ein Sackloch gebohrt sein, wobei das jeweils dem Bohrkopf zugewandte Sackloch die gleiche Funktion besitzt, wie das Sackloch innerhalb des Hohlzylinders für einen Kirschner-Draht oder Steinmann-Nagel. Das gegenüberliegende Sackloch dient in vorteilhafter Weise zum Herausnehmen der Olive oder des Stoppers nach Beendigung der Behandlung mittels des Zügeldrahtes. Denn aufgrund des durchgehenden Längsschlitzes der Olive oder des Stoppers könnte der Zügeldraht mit seinem verjüngten Teil in Nachbarschaft des Bohrkopfes aus dem Schlitz herausgleiten und die Olive oder den Stopper verlieren. Nunmehr wird zum Herausheben der Olive oder des Stoppers der Zügeldraht in Richtung des Bohrkopfes axial vorgestoßen, bis das dem Bohrkopf abgewandte Ende des verjüngten Teils in das Sackloch eingefahren ist, wodurch ein Herausgleiten des verjüngten Teils des Zügeldrahtes aus dem Schlitz der Olive oder desStoppers nicht mehr möglich ist.

Kurzbezeichnung der Figuren der Zeichnung, welche zeigen:

Figur 1 eine Ansicht eines Drahtes, beispielsweise eines Kirschner-Drahtes

Figur 2 eine um 90 Grad gedrehte Ansicht der Figur 1

Figur 3 einen Längsschnitt durch ein Haltemittel, welches ein Hohlzylinder ist

Figur 4 einen Schnitt längs der Linie A-A in Figur 3

Figur 5 eine Draufsicht von unten auf Figur 3

Figur 6 eine Draufsicht von oben auf Figur 3

Figur 7 einen Schnitt durch eine Mutter, aufgeschraubt auf das nichtpenetrierende Ende des Drahtes

Figur 8 eine Draufsicht auf Figur 7

Figur 9 eine Ansicht eines Bolzens zum Halten der Enden des Drahtes oder des Nagels auf einem Ring oder sonstigen Halteteil des Fixateurs

Figur 10 einen Schnitt längs der Linie B-B in Figur 9

Figur 11 eine Ansicht eines Nagels, insbesondere Steinmann-Nagel

Figur 12a und b zwei gegenseitig um 90 Grad gedrehte Ansichten des Bohrkopfes und des sich daran anschließenden verjüngten Teils des Nagels in Figur 11

Figur 13 ein weiteres Haltemittel, welches eine Olive oder ein Stopper ist

Figur 14 einen Schnitt durch die Olive der Figur 13 längs der Linie C-C

Figur 15 eine um 90 Grad gedrehte Ansicht der Figur 13 in Draufsicht auf den Längsschlitz und

Figur 16 eine Draufsicht aufeine Langmutter mit Sackloch und Innengewinde zum Aufschrauben auf den Gewindeteil des Drahtes oder Nagels während des Bohrvorganges.

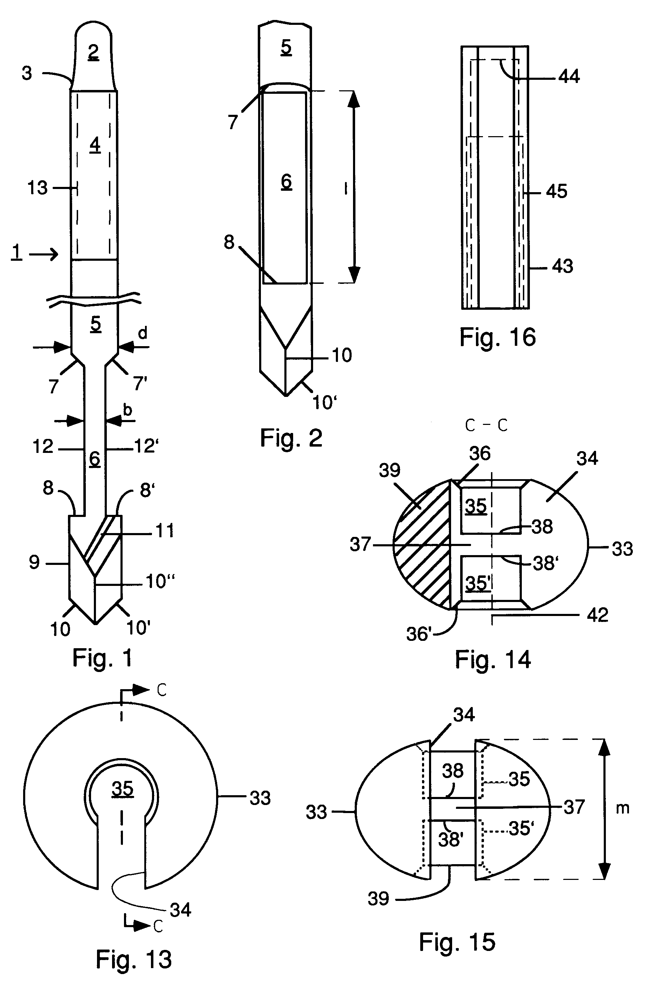

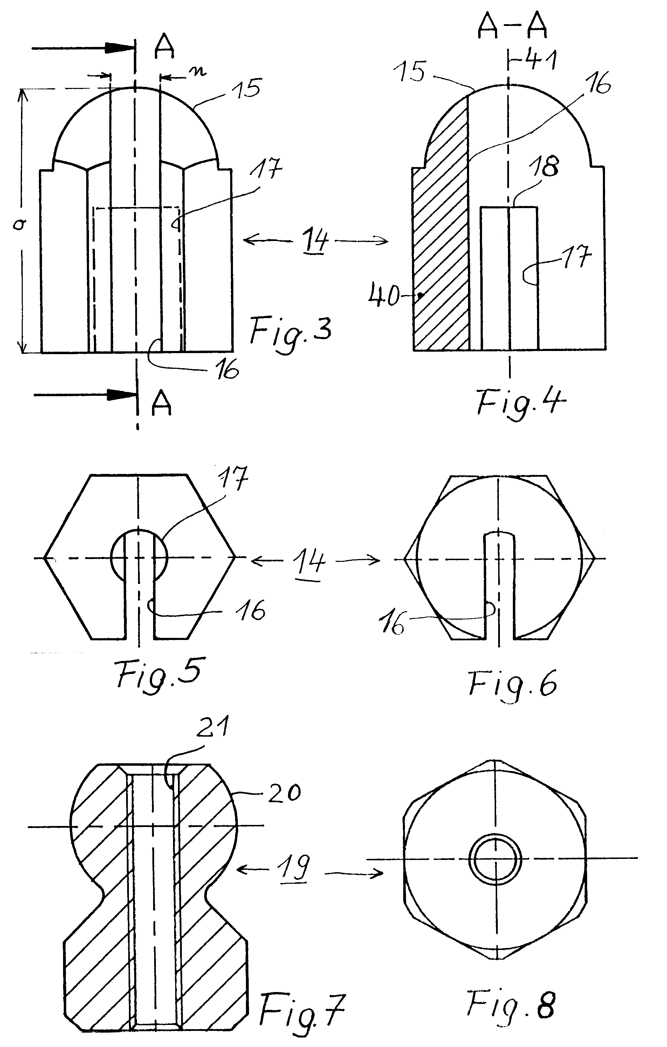

Die Figuren 1 und 2 zeigen einen Draht 1, vorzugsweise einen Kirschner-Draht, der aus einem glatten Endteil 2 besteht, an welches sich ein Gewindeteil 4 mit einem Gewinde 13 anschließt. Das glatte Endteil 2, welches zum Fassen innerhalb des Bohrfutters einer Bohrmaschine oder eines Drillbohrers dient, besitzt einen geringeren Durchmesser als das Gewindeteil 4 und geht mittels eines konusförmigen Überganges 3 in das Gewindeteil 4 über. An das Gewindeteil 4 schließt sich ein glatter, langer Schaft 5 an, der in Figur 1 unterbrochen dargestellt ist. Der Schaft 5 ist zylindrisch-glatt und geht an seinem vorderen Ende in ein verjüngtes Teil 6 über, wobei die Übergänge 7, 7′ angefast sind und schräg verlaufen. Der verjüngte Teil 6 wird durch zwei sich planparallel gegenüberstehende Flächen 12, 12′ gebildet, wobei der Abstand b der planparallelen Flächen 12, 12′ zueinander bzw. die Dicke b der verbleibenden Materialsbrücke geringer ist, als der Durchmesser d des zylindrischen Drahtes 1. In Richtung eines zu den Flächen 12, 12′ senkrecht verlaufenden Durchmessers besitzt die Materialbrücke den Durchmesser d des Schaftes 5. An das verjüngte Teil 6 schließt sich ein Bohrkopf 9 an, wobei der Übergang zwischen dem verjüngten Teil 6 und dem Bohrkopf 9 durch zwei sich gegenüberliegende Flanken 8, 8′ gebildet wird, die sich zu den Flächen 12, 12′ senkrecht erstrecken. Der Bohrkopf 9 kann eine Mehrzahl von schrägstehenden, scharfkantigen Schneiden 10, 10′, 10″ besitzen, die zum Penetrieren des Knochens beim Drehen des Drahtes 1 wie Bohrerschneiden dienen.

Des weiteren kann der Bohrkopf 9 Kanäle 11 aufweisen, die sich, von den Schneiden ausgehend, zum verjüngten Teil 6 erstrecken und in die Ausnehmungen des verjüngten Teils einmünden, die aufgrund der Flächen 12, 12′ und beim Penetrieren eines Knochens aufgrund der Knochenwandung gebildet werden. Diese Kanäle 11 können vorzugsweise schräg verlaufen und dienen zum Abtransport des Abriebs des Knochens in die durch die Flächen 12, 12′ bzw. die Verjüngung gebildeten Ausnehmungen.

Die Länge 1 (Figur 2) des verjüngten Teils 6 einschließlich der Länge des Bohrkopfes kann ungefähr ein Zwanzigstel bis ein Siebtel der Gesamtlänge des Drahtes 1 entsprechend dessen Anwendung betragen. Die Länge des Gewindeteils 4 einschließlich des Endteils 2 ist gewöhnlich etwas länger als die Länge 1 des verjüngten Teils einschließlich des Bohrkopfes.

Die Figuren 3 bis 6 zeigen ein Haltemittel 14 zum Haltern des penetrierenden transossären Endes des Drahtes 1, nachdem derselbe den Knochen oder die Weichteile durchbohrt hat und nunmehr innerhalb des Fixerteurs gehaltert werden muß. Das Haltemittel 14 ist ein Hohlzylinder, der an seinem Umfang vorzugsweise Sechskant-Schlüsselflächen besitzt und dessen ein Ende ballig-abgeflacht geformt ist, beispielsweise einen Rundkopf 15 aufweist. Der Hohlzylinder 14 weist einen durchgehenden Schlitz auf, der gemäß Figur 4 über die Längsachse 41 des Hohlzylinders 14 hinwegreicht unter Belassung eines Materialsteges 40. Die Breite n des Schlitzes 16 ist breiter als die Breite b des verjüngten Teils 6 des Drahtes 1; die Länge o des Hohlzylinders 14 ist kleiner als die Länge 1 des verjüngten Teils 6. Deshalb kann der Hohlzylinder 14 vermöge seines Schlitzes 16 auf den verjüngten Teil 6 des Nagels 1 aufgesetzt werden, und zwar dergestalt, daß das ballig-abgeflachte oder gerundete Ende 15 zum nichtpenetrierenden Ende des Drahtes 1 gerichtet ist.

Der Hohlzylinder 14 weist des weiteren, ausgehend von seinem dem Rundkopf 15 gegenüberliegenden Ende, eine Sackbohrung 17 auf, die vorzugsweise ein Fräsloch mit einer senkrechten Stirnfläche 18 ist. Das Fräsloch 17 erstreckt sich zentrisch in Richtung der Achse 41 des Hohlzylinders 14 und besitzt einen lichten Durchmesser, der größer ist als der Durchmesser des Bohrkopfes 9 bzw. des Schaftes 5 des Nagels 1, wie es am besten aus der Figur 5 zu ersehen ist, weshalb der Hohlzylinder auf dem Draht relativ verschoben werden kann, wobei das Fräsloch 17 den Bohrkopf 9 des Drahtes vollständig aufzunehmen imstande ist und in Zugstellung immer aufnimmt.

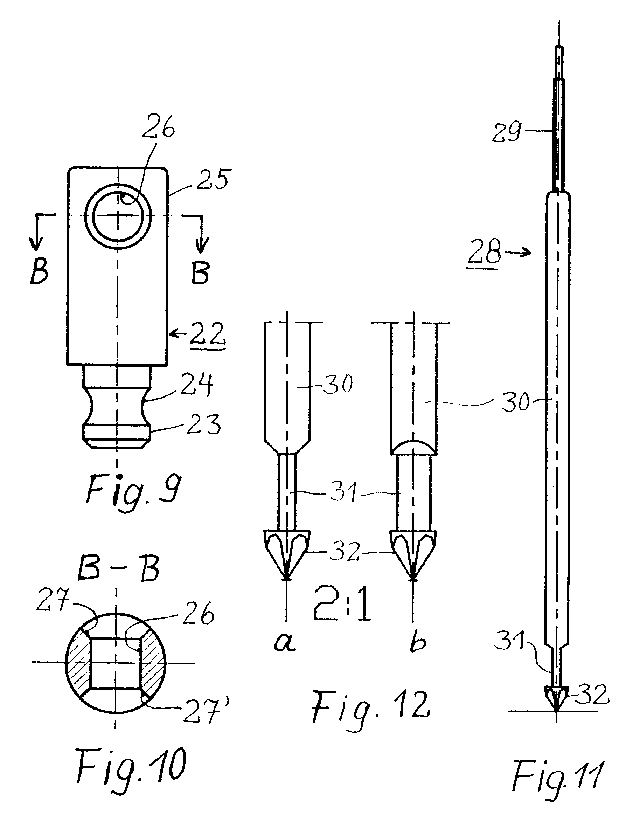

Die Figuren 7 und 8 zeigen eine Mutter 19 mit einem Kugelkopf 20 und einer Gewinde-Durchgangsbohrung 21, die zentrisch die Mutter 19 durchsetzt. Diese Mutter 19 dient zur Halterung des nichtpenetrierenden Endes des Drahtes 1 nach dem Aufschrauben auf das Gewinde 13 des Gewindeteils 4.

Die Figuren 9 und 10 zeigen einen Bolzen 22 zur Halterung des Drahtes 1 und der sich gegenüberliegenden Haltemittel 14, 19. Der Bolzen 22 besteht aus einem Bolzenschaft 25, an den ein Zapfen 23 angeformt ist, der eine umlaufende Kehle 24 besitzt. In seinem oberen Ende besitzt der Bolzenschaft 25 eine Durchgangsbohrung 26, die beidendig mit einer sphärischen Phase 27, 27′ versehen ist und die je einen Paßsitz für das ballig-abgeflachte Ende oder den Rundkopf 15 des Haltemittels 14, 19 bilden. Der Bolzen 22 ist mittels seines Zapfens 23 in geeigneter Weise innerhalb eines (nicht gezeigten) Fixateurs gehaltert.

Die Anwendung und Funktion des Drahtes 1 mitsamt den Haltemitteln 14, 19 und 22 ist folgende:

Nachdem der Draht 1 den Knochen penetriert hat, wird der Bohrkopf 9 durch das Loch 26 eines Bolzens 22 geschoben bis der verjüngte Teil 6 des Drahtes 1 die Durchgangsbohrung 26 vollständig durchragt. Dann wird auf den verjüngten Teil 6 des Drahtes 1 ein Hohlzylinder 14 mit seinem Schlitz 16 aufgesetzt, und zwar mit dem Rundkopf 15 zum Kugelsitz der Fase 27, 27' hingewandt. Nunmehr wird der Draht 1 innerhalb des Hohlzylinders 14 zurückgezogen, bis die senkrechten Flanken 8, 8' des verjüngten Teils 6 innerhalb des Fräsloches 17 auf der senkrechten Stirnfläche 18 desselben aufliegen; in dieser Stellung ist der Bohrkopf 9 vollständig in den Hohlzylinder 14 eingefahren, so daß Verletzungen durch die Spitzeoder die Schneiden des Bohrkopfes 9 ausgeschlossen sind. Das nichtpenetrierende Ende des Nagels 1 wird ebenfalls durch das Loch 26 eines weiteren Bolzens 22 geschoben und auf das hervorstehende Gewindeteil 4 einer Mutter 19 aufgeschraubt und zwar mit dem Kugelkopf zum Kugelsitz der Fase 27, 27' gerichtet. Nunmehr kann der Draht 1 durch Verdrehen der Mutter 19 gespannt werden, wobei der Draht beim Gegenhalten des Hohlzylinders 14 verdrehsicher gehaltert werden kann.

Die Figuren 11 und 12 zeigen einen Nagel 28, vorzugsweise ein SteinmannNagel, bestehend aus einem Gewindeteil 29, an den sich ein langer, glatter Schaft 30 anschließt, der am penetrierenden Ende des Nagels 28 in einen verjüngten Schaftteil 31 übergeht, an den sich ein Bohrkopf 32 anschließt. Die Ausgestaltung und die Fuktion des Nagels 28 entspricht derjenigen des oben beschriebenen Drahtes 1.

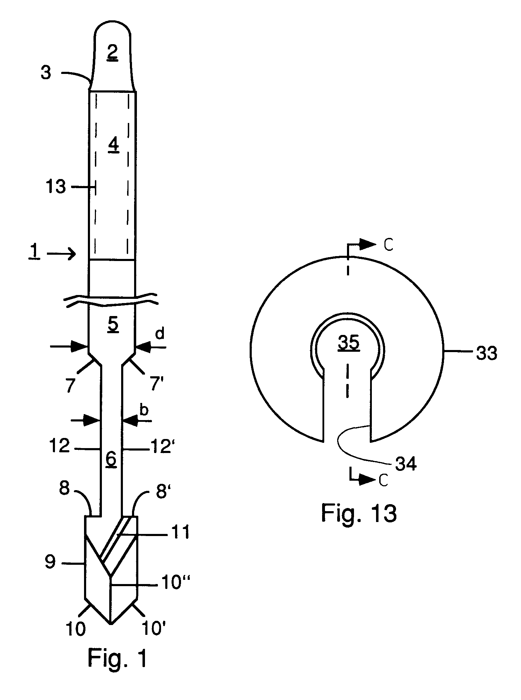

Die Figuren 13 und 14 zeigen eine Olive 33, die im Zusammenspiel mit einem Zügeldraht, der wie der Draht 1 der Figuren 1 und 2 gestaltet sein kann, Verwendung findet. In diesem Fall ist das nichtpenetrierende Ende desDrahtes in einem Bolzen, wie oben beschrieben, gehaltert; das transossär penetrierende Ende kann mittels der Olive ebenfalls innerhalb eines Bolzens oder aber im Körper am Knochen direkt zur Zügelung gehaltert sein.

Die Olive 33 ist prinzipiell gleichermaßen geformt wie der Hohlzylinder 10 und besitzt einen durchgehenden Längsschlitz 34, der über die Mittelachse 42 der Olive 33 unter Belassung eines Materialsteges 39 hinweggreicht. Zentrisch in die Olive 33 in Richtung ihrer Mittelachse 42 sind von jeder Oberfläche ausgehend ein Fräsloch 35, 35' gebohrt, die zwischen sich einen Steg 37 belassen und die senkrecht zur Bohrung gerichtete Stirnflächen 38,38' aufweisen. Des weiteren können die nach außn weisenden Enden der Fräslöcher 35, 35' mit einer umlaufenden trichterförmig gestalteten Fase 36, 36' versehen sein.

Das eine Fräsloch der Olive 33 übernimmt die Funktion des Fräsloches 17 des Hohlzylinders 14 der Figuren 3 und 4. Das andere Fräsloch dient zum sicheren Ergreifen und Herausheben der Olive 33 mittels des Zügeldrahtes aus der Halterung, beispielsweise aus den Weichteilen. Zu diesem Zweck wird der Draht 1 in Richtung der Olive 33 vorgeschoben, bis das vom penetrierenden Ende abgewandte Ende des verjüngten Teils des Zügeldrahtes in das Fräsloch innerhalb der Olive 33 einfährt. Nunmehr kann die Olive 33 mittels des Zügeldrahtes abgehoben werden, ohne daß dieselbe aufgrund des Schlitzes 34 innerhalb der Olive 33 vom verjüngten Teil des Zügeldrahtes abrutschen kann.

Das Haltemittel kann auch in Form eines Stoppers ausgestaltet sein, der prinzipiell wie die vorbeschriebene Olive gestaltet ist, aber eine kleine Kugel ist, die vorzugsweise einen Durchmesser zwischen 4 bis 6 mm besitzt.

Figur 16 zeigt eine Langmutter 43, die vorzugsweise sechskantig ist und die ein Sackloch 44 aufweist, welches ein Innengewinde 45 aufweist. Die Langmutter dient zum Aufschrauben auf den Gewindeteil 4 oder 29 des Drahtes 1 oder Nagels 28, um das Gewinde 13 desselben während des Bohrvorganges vor Beschädigungen zu schützen. Die Langmutter 43 wird auf das Gewinde 13 des Gewindeteils 4, 29 aufgeschraubt und innerhalb des Bohrfutters einer Bohrmaschine gehaltert, wodurch der Draht 1 oder Nagel 28 rotatorisch mitgenommen wird. Nachdem der Draht 1 oder Nagel 28 den Knochen und die Weichteile penetriert hat und das penetrierende Ende des Drahtes 1 oder Nagels 28 gehaltert ist, wird die Langmutter 43 abgeschraubt und der Gewindeteil des Drahtes 1 oder Nagels 28 wie vorgesehen mittels einer Mutter 19 ebenfalls gehaltert.

Wenn der Bohrkopf das Haltemittel nach dem transossären Durchgang durchstoßen sollte, kann in allgemeiner Form auf den Bohrkopf und/oder das Haltemittel eine in der Gestaltung angepaßte Schutzkappe aufgesetzt sein, die die Form einer Überwurfmutter, vorzugsweise mit Konus, besitzen kann, die auf ein Außengewinde des Bohrkopfes und/oder des Haltemittels aufgeschraubt ist.

Gewerbliche Anwendbarkeit:

Der Draht oder Nagel mit Haltemittel dient vorzugsweise zur Anwendung innerhalb eines Fixateurs, um im funktionalen Zusammenspiel Knochenabschnitte und/oder Knochenfragmente einzurichten, zu lenken und zu zügeln.

Liste der Bezugszeichen:

| 1 | Kirschner-Draht |
| 2 | glattes Endteil |

| 3 | konusförmiger Übergang |
|---|---|
| 4 | Gewindeteil |
| 5 | glatter Schaft |
| 6 | verjüngter Schaftteil |
| 7, 7′ | angefaste Übergänge |
| 8, 8′ | Flanken |
| 9 | Bohrkopf |
| 10, 10′, 10″ | Schneiden |
| 11 | Kanal |
| 12., 12′ | planparallele Flächen |
| 13 | Gewinde |
| 14 | Mutter |
| 15 | Rundkopf |
| 16 | Schlitz |
| 17 | Fräsloch |
| 18 | senkrechte Stirnfläche |
| 19 | Mutter |
| 20 | Kugelkopf |
| 21 | Gewinde-Durchgangsbohrung |
| 22 | Bolzen |
| 23 | Zapfen |
| 24 | umlaufende Kehle |
| 25 | Bolzenschaft |
| 26 | Durchgangsbohrung |
| 27, 27′ | sphärische Fasen |
| 28 | Nagel |
| 29 | Gewindeteil |
| 30 | glatter Schaft |
| 31 | verjüngter Schaftteil |
| 32 | Bohrkopf |
| 33 | Olive |
| 34 | Schlitz |
| 35, 35′ | Sacklöcher |
| 37 | Steg |
| 38, 38′ | senkrechte Stirnflächen |
| 39, 40 | Materialbrücken |
| 41, 42 | Mittelachsen |
| 43 | Langmutter |
| 44 | Sackloch |
| 45 | Innengewinde |

**Patentansprüche**

1. Draht (1) oder Nagel (28) mit Haltemittel (14,33), insbesondere für einen Fixateur zum Einrichten, Befestigen und Regulieren der Spannlage von Knochenabschnitten, wie Kirschner-Draht oder Zügeldraht oder Steinmann-Nagel, mit einem Gewinde- und/oder Halterungsteil an beiden Enden zum Halten des Drahtes oder Nagels innerhalb mindestens eines Bolzens (22) des Fixateurs mittels Muttern (19) und/oder der Haltemittel (14,33), die auf die Enden des Drahtes oder Nagels lösbar aufgesetzt sind, gekennzeichnet durch folgende Merkmale:

   a) der Draht (1) oder Nagel (28) besitzt einen glatten Schaft (5,30), der am penetrierenden Ende einen Bohrkopf (9,32) aufweist, an den sich ein gegenüber dem Schaft verjüngtes Teil (6,31) mit planparallelen Begrenzungsflächen (12,12′) anschließt, dessen Breite (b) geringer ist als der Durchmesser (d) des Schaftes

   b) das Haltemittel (14,33) weist einen durchgehenden Schlitz (16,34) auf, der breiter ist als die Breite (6) des verjüngten Teils (6,31) des Schaftes (5,30), wobei die Länge des Haltemittels (14,33) kleiner ist als die Länge (1) des verjüngten Teils des Schaftes zum Aufsetzen des Haltemittels (14,33) auf den verjüngten Teil (6,31) des Schaftes (5,30)

c) innerhalb des Haltemittels (14,33) ist zentrisch in Richtung des Schlitzes (16,34) von dem dem penetrierenden Ende (15) gegenüberliegenden Ende des Haltemittels (14,33) ausgehend mindestens eine Sackbohrung (17,35,35') angeordnet, deren Durchmesser größer ist als der Durchmesser (d) des Schaftes (5,30)

d) in Zugstellung des Drahtes (1) oder Nagels (28) innerhalb des Fixateurs ist der Bohrkopf (9,32) vollständig innerhalb des Haltemittels (14,33) aufgenommen.

2.  Draht oder Nagel nach Anspruch 1, dadurch gekennzeichnet,
    daß der verjüngte Teil (6,31) des Schaftes (5,30) an dem dem Bohrkopf (9,32) zugewandten Ende zu den planparallelen Begrenzungsflächen (12,12') senkrecht gerichtete Flanken (8,8') aufweist und daß die Sackbohrung innerhalb des Haltemittels (14) ein Sackloch (17) mit senkrecht zur Bohrung gerichteter Stirnfläche (18) aufweist, wobei mindestens das penetrierende Ende (15) des Haltemittels (14,33) mindestens am Rand ballig-abgeflacht oder gerundet ist.

3.  Draht oder Nagel nach Anspruch 1 oder 2, dadurch gekennzeichnet,
    daß der verjüngte Teil (6,31) des Schaftes (5,30) an seinem dem Bohrkopf (9,32) abgewandten Ende schräg zu den planparallelen Begrenzungsflächen (12,12') verlaufende Flanken (7,7') aufweist, die in den Schaft (5,30) übergehen.

4.  Draht oder Nagel nach Anspruch 1, dadurch gekennzeichnet,
    daß derselbe an seinem nichtpenetrierenden Ende ein Gewinde (13) aufweist, dessen Länge zwischen ein Zwanzigstel bis ein Viertel der Länge des gesamten Drahtes (1) oder Nagels (28) beträgt und daß auf dem Gewinde eine Mutter (19) angeordnet ist, die einen Kugelkopf (20) aufweist als Paßsitz innerhalb eines entsprechenden Sitzes (27,27') einer Bohrung (26) des Bolzens (22).

5.  Draht oder Nagel nach Anspruch 1, dadurch gekennzeichnet,
    daß der Bohrkopf (9) schräg zueinander geneigte Schneiden (10,10',10") aufweist und ausgehend von wenigstens einer Schneide (10, 10', 10"), mindestens einen Kanal (11) besitzt, der zu dem verjüngten Teil (6) des Schaftes (5) jeweils zu den aufgrund der planparallelen Flächen (12, 12') gebildeten Ausnehmungen führt, wobei die Kanäle (11) schräg oder spiralförmig geführt sind.

6.  Draht, insbesondere Zügeldraht, oder Nagel nach Anspruch 1, dadurch gekennzeichnet,
    daß das Haltemittel für das penetrierende Ende des Drahtes entweder ein Hohlzylinder (14) ist oder die Form einer Olive (33) aufweist, deren Dicke (m) in axialer Richtung des Schlitzes (34) bzw. der Sackbohrungen (35,35') kleiner als die Länge des verjüngten Teils des Schaftes des Drahtes oder des Nagels ist zum Aufsetzen der Olive mittels des Schlitzes auf den verjüngten Teil.

7.  Draht oder Nagel nach Anspruch 1 oder 6, dadurch gekennzeichnet,
    daß der Hohlzylinder oder die Olive (33) unter Belassung eines Steges (37) zentrisch von beiden Seiten ausgehend in Richtung des Schlitzes (34) je ein Sackloch (35,35') aufweist, deren Durchmesser größer ist als der Durchmesser des Schaftes bzw. des Bohrkopfes des Drahtes oder Nagels.

8.  Draht oder Nagel nach Anspruch 7, dadurch gekennzeichnet,
    daß die Sacklöcher (35,35') zu Beginn je eine umlaufende, trichterförmige Fase (36,36') aufweisen.

9.  Draht oder Nagel nach Anspruch 1 oder 2, dadurch gekennzeichnet,
    daß das nichtpenetrierende Draht- oder Nagelende des Drahtes (1) oder Nagels (28) ein wenigstens auf den Gewindekern des Gewindeteils (4) abgedrehtes, glattes Endteil (2) aufweist.

10. Draht oder Nagel nach Anspruch 9, dadurch gekennzeichnet,
    daß auf den Gewindeteil (4,29) des Drahtes (1) oder Nagels (28) eine Langmutter (43) mit einem Sackloch (44) mit Innengewinde (45) aufschraubbar ist zum Schutz des Gewindes (13) des Gewindeteils während des Bohrvorganges.

11. Draht oder Nagel nach Anspruch, dadurch gekennzeichnet,
    daß auf den Bohrkopf und/oder das Haltemittel eine Schutzkappe aufgesetzt ist, die die Form einer Überwurfmutter, vorzugsweise mit Konus, besitzt, die auf ein Außengewinde des Bohrkopfes und/oder des Haltemittels aufgeschraubt ist.

Fig. 1

Fig. 2

Fig. 16

Fig. 13

Fig. 14

Fig. 15

Fig.3

Fig.4

Fig.5

Fig.6

Fig.7

Fig.8

Fig. 9

Fig. 10

Fig. 12

Fig. 11

Fig. 1

Fig. 13

| Europäisches Patentamt | EUROPÄISCHER RECHERCHENBERICHT | Nummer der Anmeldung |

EP   91 10 9822

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A,D | WO-A-8 810 099 (SCHEWIOR) <br> * Seite 18, Zeile 23 - Seite 19, Zeile 11; Abbildungen 1,11 * <br> --- | 1 | A61B17/60 |
| A | FR-A-1 046 555 (MEROU) <br> * Seite 1, Zeile 31 - Seite 2, Zeile 21; Abbildungen 2,5,6 * <br> --- | 1 | |
| A | GB-A-2 031 731 (KRONNER) <br> * Seite 4, Zeile 40 - Zeile 71; Abbildung 17 * <br> ----- | 1 | |
| | | | **RECHERCHIERTE SACHGEBIETE (Int. Cl.5 )** <br><br> A61B |

Der vorliegende Recherchenbericht wurde für alle Patentansprüche erstellt

| Recherchenort | Abschlußdatum der Recherche | Prüfer |
|---|---|---|
| DEN HAAG | 30 OKTOBER 1991 | MOERS R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.............................................................
& : Mitglied der gleichen Patentfamilie, übereinstimmendes Dokument

EPO FORM 1503 03.82 (P0403)